# EUROPEAN PATENT APPLICATION

(11) **EP 4 772 143 A1**
(43) Date of publication of application: **08.07.2026**
(21) Application number: 23950889.8
(22) Date of filing: 01.09.2023
(51) Int. Cl.: A61F 2/14, A61L 27/16, C08F 265/06, C08F 220/20, C08F 2/00, C08F 2/48

(54) **METHOD FOR MANUFACTURING ARTIFICIAL CORNEA USING INTERFACIAL ADHESION BETWEEN OPTICAL UNIT AND SUPPORT UNIT HAVING DIFFERENT PROPERTIES**

(71) Applicant: Te Bios Co., Ltd, Cheongju-si, Chungcheongbuk-do 28160 (KR)
(72) Inventor: JEONG, Do Sun, Cheongju-si, Chungcheongbuk-do 28162 (KR); KIM, Tae Hyun, Cheonan-si, Chungcheongnam-do 31091 (KR); KIM, Ja Rok, Cheongju-si, Chungcheongbuk-do (KR); PARK, Jae Hong, Cheongju-si, Chungcheongbuk-do 28160 (KR)
(74) Representative: Vossius & Partner Patentanwälte Rechtsanwälte mbB
(86) International application number: PCT/KR2023/013089
(87) International publication number: WO 2025/048014

(57) **Abstract**

The present invention relates to a method for manufacturing an artificial cornea exhibiting excellent physical properties by stably adhering an interface between an optical unit and a support unit by means of an artificial cornea mold and UV dose modulation. According to the present invention, an optical unit is manufactured for one to four hours using low-dose UV in an artificial cornea mold so as to stably adhere an interface between an optical unit and a support unit, thereby manufacturing an artificial cornea in which the optical unit and the support unit are integrated with each other. The artificial cornea manufactured by the manufacturing method has a consistent spherical shape, which is convenient for centering and increases surgical success rates during surgery, the central part of the optical unit exhibits uniform and excellent physical properties to further increase physical safety in the human body, and the boundary surface between the optical unit and the support unit exhibits about 100 times more excellent physical properties compared to existing intraocular pressure, thus allowing early prevention of the optical unit and the support unit separating due to intraocular pressure in the human body. In addition, the manufacturing method can improve the appearance of a patient implanted with an artificial cornea and reduce complications and side effects caused by adhesive material.

## Description

### Technical Field

Aspects of the present disclosure relate to a method for manufacturing an artificial cornea that can exhibit excellent physical properties by stably adhering an interface between an optical unit and a support unit.

### Background Art

An artificial cornea is a product used for patients with damaged corneas, and may require an optical unit (core) for securing a visual field and a support unit (skirt) for stably attaching the optical unit to an eye. The optical unit may be colorless and transparent, having a visible light transmittance of 99% or more, and may serve to transmit light by replacing the damaged cornea. The support unit may be formed of an opaque white cell scaffold structure and may serve to fix the artificial cornea to the eye through autologous cell proliferation.

In current technologies, a method capable of adhering the optical unit and the support unit in a simple manner does not exist. Thus, methods such as fixing the cornea by inserting it between a metallic material and a plastic, or coupling the optical unit and the support unit using a nylon suture, have been used.

However, such methods involve complicated manufacturing processes, which may increase the size of the product. This can result in an appearance of the eye that differs from that of a normal person even after corneal surgery, which may cause a decrease in the self-esteem and sociability of the patient.

In addition, such methods use metals and sutures, which are not materials typically required for an artificial cornea, thereby presenting a problem in that they may not be free from infections caused by foreign substances after surgery.

To solve this problem, various methods such as a plasma technique and a graft polymerization method for interfacial adhesion between the optical unit and the support unit have been proposed in academia. However, these methods may only provide slight adhesiveness between the interfaces and may exhibit properties degraded compared to the individual physical properties of the optical unit and the support unit, indicating limitations in their application to actual products.

Therefore, there is a need to develop a technology applicable to an artificial cornea capable of adhering the optical unit and the support unit without using other materials, thereby improving the aesthetic appearance for the patient and reducing complications and side effects caused by adhesive materials.

Additionally, an artificial cornea procedure may involve removing the clouded cornea by making a semicircular incision in the anterior lamella of the cornea and then removing a 3.5 mm to 5.5 mm disc-shaped portion of the posterior lamella of the cornea. After adjusting the central portion of the posterior lamella of the cornea and the central portion of the artificial cornea to position them as centrally as possible, the anterior lamella may be covered, and a visual field may be secured through the incision in the anterior lamella. During this process, if the artificial cornea is not positioned exactly in the center, it may not match the underlying cornea according to the designed radius of curvature, which can lead to secondary side effects. Furthermore, light may not be refracted as intended, potentially preventing an image from forming on the lens. In more severe cases, there is a risk that the artificial cornea may leak through the excised anterior lamella.

Accordingly, there is also a demand for the development of technology to stabilize the shape so that the artificial cornea can be used as designed.

### Prior Art Documents

*Patent Documents* (Patent Document 1) KR 10-2016-0140687 (2016-10-27).

### Technical Problem

As a result of earnest efforts to provide a method for manufacturing an artificial cornea capable of providing a stabilized shape and excellent physical properties while adhering an optical unit and a support unit without using other materials, the present inventors have completed the present disclosure by confirming that an artificial cornea having excellent physical properties may be manufactured by providing adhesiveness through the formation of a double network between the interface of the optical unit and the support unit when only a part of the optical unit is manufactured by photopolymerization and the remaining part of the optical unit is manufactured by thermal polymerization together with the support unit. Accordingly, an object of some embodiments of the present disclosure may be to provide a method for manufacturing an artificial cornea capable of exhibiting a stabilized shape and excellent physical properties by stably adhering an interface between an optical unit and a support unit.

### SUMMARY OF INVENTION

Aspects of the present disclosure may provide a method for manufacturing an artificial cornea consisting of an optical unit and a support unit, and the method may comprise: i) manufacturing the optical unit by irradiating a first mixed solution comprising an acrylic monomer, a photoinitiator, a crosslinking agent, methanol, and distilled water with an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine to the manufactured optical unit and performing thermal polymerization at 30°C to 100°C for 30 minutes to 2 hours.

In some aspects, the acrylic monomer of step i) may be mixed in an amount of 70 wt% to 90 wt%, the photoinitiator may be mixed in an amount of 0.1 wt% to 3 wt% based on the monomer, and the crosslinking agent may be mixed in an amount of 0.1 wt% to 0.3 wt% based on the monomer.

In some aspects, the acrylic monomer of step ii) may be mixed in an amount of 15 wt% to 35 wt%, the crosslinking agent may be mixed in an amount of 1 wt% to 3 wt% based on the monomer, the thermal initiator may be mixed in an amount of 3 wt% to 6 wt% based on the monomer, and the N,N,N',N'-tetramethylethylenediamine may be mixed in an amount of 2 wt% to 4 wt% based on the monomer.

In some aspects, the acrylic monomer may be at least one selected from the group consisting of methyl methacrylate, 2-hydroxyethyl methacrylate, trimethylolpropane triacrylate, 1,4-butanediol diacrylate, ethylhexyl acrylate, glycidyl methacrylate, ethylene glycol dimethacrylate, and 1,6-hexanediol diacrylate.

In some aspects, the photoinitiator of step i) may be at least one selected from the group consisting of 2-hydroxy-4'-(2-hydroxyethoxy)-2-methylpropiophenone, 1-hydroxy-cyclohexylphenyl-ketone, and bis(2,4,6-trimethylbenzoyl)-phenylphosphineoxide.

In some aspects, the crosslinking agent of step i) may be at least one selected from the group consisting of pentaerythritol tetraacrylate, pentaerythritol triacrylate, N,N'-methylenebisacrylamide, and ethylene glycol dimethacrylate (EGDMA).

In some aspects, the distilled water and the methanol of step i) may be mixed in a weight ratio of 75 to 95 : 5 to 25.

In some aspects, the optical unit may be manufactured by only 80% to 99% through the photopolymerization in step i).

In some aspects, the distilled water and the dimethylformamide of step ii) may be mixed in a weight ratio of 70 to 90 : 10 to 30.

In some aspects, the crosslinking agent of step ii) may be at least one selected from the group consisting of pentaerythritol tetraacrylate, pentaerythritol triacrylate, N,N'-methylenebisacrylamide, and ethylene glycol dimethacrylate (EGDMA).

In some aspects, the thermal initiator of step ii) may be ammonium persulfate and/or potassium persulfate.

Additionally, aspects of the present disclosure may provide an artificial cornea manufactured by the manufacturing method.

In some aspects, a tensile strength of an interface between the optical unit and the support unit of the artificial cornea may be 100 KPa to 200 KPa.

In some aspects, an overall diameter of the artificial cornea may be 7 mm to 9 mm, a diameter of the optical unit may be 3 mm to 6 mm, and an apical thickness may be 0.4 mm to 0.6 mm.

In some aspects, a radius of curvature of the artificial cornea may be 7.5 mm to 8.0 mm on an anterior surface and 6.0 mm to 7.0 mm on a posterior surface.

Furthermore, aspects of the present disclosure may provide a method for manufacturing an artificial cornea consisting of an optical unit and a support unit, and the method may comprise: i) dispensing a first mixed solution comprising an acrylic monomer, a photoinitiator, a crosslinking agent, methanol, and distilled water into a lower mold, coupling an upper mold, and manufacturing the optical unit by irradiating an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine into the lower mold where the optical unit is formed, and performing thermal polymerization at 30°C to 100°C for 30 minutes to 2 hours.

In some aspects, step ii) may be continuously performed without separating the upper mold and the lower mold.

### Advantageous Effects

According to some embodiments of the present disclosure, an optical unit may be manufactured for 1 hour to 4 hours using a low-dose UV, so that an interface between the optical unit and a support unit may be stably adhered, thereby manufacturing an artificial cornea in which the optical unit and the support unit may be integrated. The artificial cornea manufactured by the manufacturing method may have a consistent spherical shape, making centering convenient and potentially increasing a surgical success rate during surgery, a central portion of the optical unit may exhibit uniform and excellent physical properties to further increase physical safety in a human body, and a boundary surface between the optical unit and the support unit may exhibit excellent physical properties of about 100 times that of existing intraocular pressure to early prevent separation of the optical unit and the support unit due to intraocular pressure in the human body. In addition, through the manufacturing method, aesthetics of a patient who has received an artificial cornea transplant may be improved, and complications and side effects caused by adhesive materials may be reduced.

### Brief Description of the Drawings

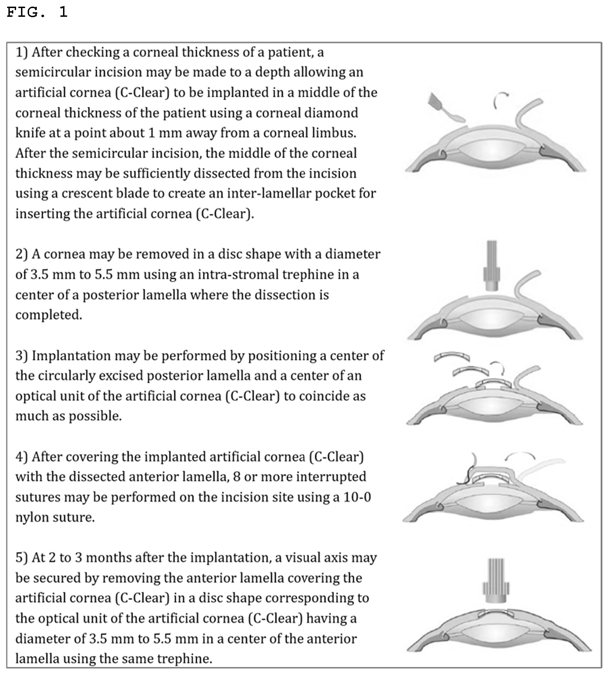

FIG. 1 may illustrate an artificial cornea surgical procedure. FIG. 2a may illustrate an optical unit of an artificial cornea manufactured by irradiating an 8 W UV for 2 hours.
FIG. 2b may illustrate that degrees of polymerization of a central portion (80% to 99%) and a peripheral portion (1% to 20%) of an optical unit of an artificial cornea manufactured by irradiating an 8 W UV for 2 hours may be formed differently. The central portion of the optical unit may be 100% completely polymerized, and the peripheral portion may be polymerized only at an oligomer level.
FIG. 3 may illustrate an artificial cornea manufactured by irradiating an 8 W UV for 2 hours.
FIG. 4a may illustrate a shape change of an optical unit according to a UV irradiation time. FIG. 4b may illustrate an external shape of an artificial cornea manufactured by performing photopolymerization for 4 hours or more.
FIG. 5a may illustrate an optical unit of an artificial cornea manufactured by irradiating a 40 W high-energy UV for 2 hours. FIG. 5b may illustrate an artificial cornea manufactured by irradiating a 40 W high-energy UV for 2 hours.
FIG. 6 may illustrate an artificial cornea in which an optical unit and a support unit may be adhered using an oxygen plasma.
FIG. 7a may illustrate an SEM image of an artificial cornea manufactured by irradiating an 8 W UV for 2 hours. It may be a structure in which beads are woven, and a surface shape may be roughened, thereby increasing cell adhesion.
FIG. 7b may illustrate a BCS Technology (Beads Chain Scaffold) used for a support unit of an artificial cornea according to some embodiments.
FIG. 8 may illustrate an SEM image of an artificial cornea manufactured by irradiating a 40 W UV for 2 hours.
FIG. 9 may illustrate stress-strain (S-S) curves of artificial corneas manufactured independently by manufacturing methods of UV polymerization + thermal polymerization, UV polymerization + oxygen plasma, and UV polymerization.
FIG. 10a may illustrate initial elastic moduli and maximum tensile strengths of artificial corneas manufactured independently by manufacturing methods of UV polymerization/thermal polymerization, UV polymerization/oxygen plasma, and UV polymerization.
FIG. 10b may illustrate a maximum tensile strength of an artificial cornea manufactured according to a manufacturing method of some embodiments. It may be confirmed that a physical strength of an interface may have a physical property of an interface between an optical unit and a support unit (170 KPa) superior to a strength of the support unit through an interfacial bonding between the optical unit (1390 KPa) and the support unit (40 KPa).
FIG. 10c may illustrate a maximum tensile strength of an artificial cornea on which photopolymerization may be performed using a 40 W high-energy UV. It may be confirmed that a maximum tensile strength of an interface may be about 10 KPa, indicating that a weak bonding may occur.
FIG. 10d may illustrate maximum tensile strengths of five optical units of an artificial cornea using a 40 W high-energy UV (Sample 1: 1416 KPa, Sample 2: 1046 KPa, Sample 3: 1532 KPa, Sample 4: 957 KPa, Sample 5: 1042 KPa). It may be confirmed that a standard deviation may be very large.
FIG. 10e may illustrate maximum tensile strengths of five optical units of an artificial cornea manufactured according to a manufacturing method of some embodiments (Sample 1: 1398 KPa, Sample 2: 1351 KPa, Sample 3: 1431 KPa, Sample 4: 1356 KPa, Sample 5: 1344 KPa). It may be confirmed that a uniform tensile strength may be exhibited.
FIG. 11a may illustrate a mold for manufacturing an artificial cornea to which an artificial cornea manufacturing method according to some embodiments may be applied.
FIG. 11b may illustrate a mold for manufacturing an artificial cornea to which an artificial cornea manufacturing method according to some embodiments may be applied. A first hole 11 may be formed in a central portion of an upper mold 10, a solution 12 for forming an optical unit may be inserted into the first hole 11, and a second hole 13 for introducing a solution for forming a support unit may be formed in a peripheral portion of the upper mold 10. In addition, a plurality of lower fitting protrusions 21 for coupling with the upper mold 10 may be formed on a lower mold 20.
FIG. 11c may illustrate a mold for manufacturing an artificial cornea to which an artificial cornea manufacturing method according to some embodiments may be applied. A first hole 11 may be formed in a central portion of an upper mold 10, a solution 12 for forming an optical unit may be inserted into the first hole 11, and a second hole 13 for introducing a thermosetting solution for forming a support unit may be formed in a peripheral portion of the upper mold 10.
FIG. 11d may illustrate a mold for manufacturing an artificial cornea to which an artificial cornea manufacturing method according to some embodiments may be applied. A plurality of lower fitting protrusions 21 for coupling with an upper mold 10 may be formed on a lower mold 20.
FIG. 12 may illustrate an artificial cornea manufacturing sequence using an artificial cornea manufacturing mold according to some embodiments.
FIG. 13 may illustrate an external shape of an artificial cornea manufactured according to some embodiments.
FIG. 14 may illustrate a detailed structure of an artificial cornea manufactured according to some embodiments (A: overall diameter, B: optical unit diameter, C: apical thickness, D: radius of curvature on an anterior surface, E: radius of curvature on a posterior surface).
FIG. 15 may illustrate results of confirming an in vitro cell proliferation effect of an artificial cornea manufactured according to some embodiments.
FIG. 16 may illustrate results of confirming an in vivo biocompatibility and cell proliferation effect of an artificial cornea manufactured according to some embodiments.
FIG. 17 may illustrate results of implanting an artificial cornea manufactured according to some embodiments into a rabbit.
FIG. 18 may illustrate results of implanting an artificial cornea manufactured according to some embodiments into a primate.

### DETAILED DESCRIPTION

To couple an optical unit and a support unit, they may generally be integrated using a bioadhesive or a suture. However, since an artificial cornea may have characteristics of a soft tissue having flexibility rather than being hard, when the bioadhesive is used, a bonded portion may have a characteristic of becoming partially unstable, and even when the suture is used, there may be a concern that the artificial cornea may be torn due to a narrow surface area of the suture. In addition, when an additional material is used for fusion, an infection caused by the additional material and an unnecessary surgical procedure may be added.

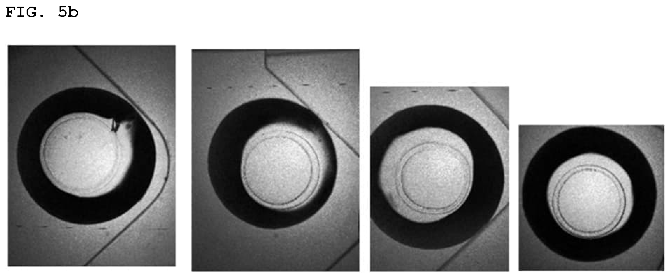

Furthermore, when the artificial cornea is manufactured using high-energy UV, a central portion of the artificial cornea may not coincide (FIG. 5b). This may be because, during photopolymerization using a high-energy UV lamp, a reaction may occur unevenly, thereby causing a deformation in a shape of the optical unit.

Accordingly, to fuse the optical unit and the support unit, polymerization conditions for the optical unit may be changed, and the artificial cornea may be manufactured by simultaneously polymerizing the optical unit and the support unit during a polymerization process. In some embodiments, degrees of polymerization of a central portion (80% to 99%) and a peripheral portion (1% to 20%) of the optical unit may be controlled differently by irradiating light of weak intensity. The central portion of the optical unit to which the light of weak intensity is intensively exposed may have a degree of polymerization of 100%, so that transparency and physical properties for securing a visual field may not be affected, and the peripheral portion relatively less exposed to the light may be polymerized only at an oligomer level to have liquid properties. Thereafter, when a support unit solution is introduced, it may be mixed with the peripheral portion of the optical unit, and the peripheral portion of the optical unit may be additionally thermally polymerized together with the support unit due to a thermal initiator (APS) present in the support unit solution. Ultimately, the peripheral portion of the optical unit and the support unit solution may be simultaneously polymerized and fused in a double network form, thereby forming the artificial cornea.

When the artificial cornea is manufactured by the method according to some embodiments, a boundary surface between the optical unit and the support unit may be bonded, an interfacial tensile strength may be high, and excellent physical properties may be exhibited compared to a case of adhering with oxygen plasma, which is a general method for bonding different interfaces. In addition, the support unit according to some embodiments may have a form in which beads are woven, so that a surface shape may be rough, and porosity may be provided, thereby being optimized for cells to grow. In particular, since the artificial cornea manufactured by the method according to some embodiments may be adhered to an eye using a cell scaffold without using the suture, a secondary infection and a foreign body reaction caused by the suture may be minimized. Furthermore, since autologous cells infiltrate into the cell scaffold and grow by themselves, an immune rejection response may be minimized.

In addition, when the artificial cornea is manufactured by the method according to some embodiments, it may be confirmed that the central portion of the artificial cornea coincides. This may be because UV with a weak energy is irradiated to the central portion as a whole, so that the reaction may be uniformly formed.

On the other hand, when the optical unit and the support unit are separately polymerized using high-energy UV of 40 W, the optical unit may be 100% polymerized and may not be fused with the support unit solution, so that it may be confirmed on an actual SEM image that the interface between the optical unit and the support unit may not be bonded, and the interfacial tensile strength and physical properties may be very low.

Accordingly, aspects of the present disclosure may provide a method for manufacturing an artificial cornea consisting of an optical unit and a support unit, and the method may comprise: i) manufacturing the optical unit by irradiating a first mixed solution comprising an acrylic monomer, a photoinitiator, a crosslinking agent, methanol, and distilled water with an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine to the manufactured optical unit and performing thermal polymerization at 30°C to 100°C for 30 minutes to 2 hours.

Preferably, the photopolymerization of step i) may be performed by irradiating a UV ray of 7 W to 10 W and 350 nm to 380 nm for 2 hours to 3 hours, and the thermal polymerization of step ii) may be performed at 30°C to 40°C for 50 minutes to 70 minutes.

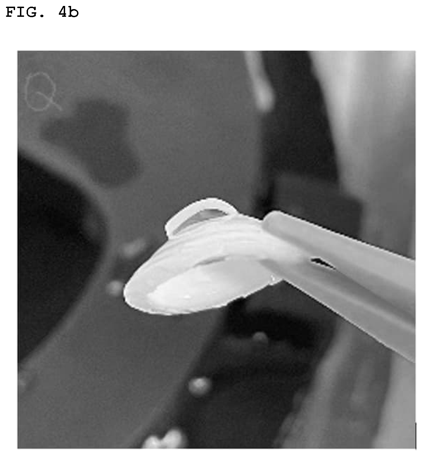

In step i), when the UV ray is irradiated for less than 1 hour, a polymer may not be formed, so that sufficient physical properties of the optical unit may not be maintained. **A target thickness of the optical unit may be 0.5 mm, which is most similar to a human body, but when the photopolymerization is performed for less than 1 hour, a phenomenon in which the thickness decreases to 0.3 mm or less may be exhibited.** On the other hand, when irradiated for 4 hours or more, all of the optical unit may be polymerized (solidified), so that adhesion between the optical unit and the support unit may not be achieved (FIG. 4b).

The N,N,N',N'-tetramethylethylenediamine of step ii) may be included to control a reaction rate of the interface.

In some aspects, the acrylic monomer of step i) may be mixed in an amount of 70 wt% to 90 wt%, the photoinitiator may be mixed in an amount of 0.1 wt% to 3 wt% based on the monomer, and the crosslinking agent may be mixed in an amount of 0.1 wt% to 0.3 wt% based on the monomer, and the acrylic monomer of step ii) may be mixed in an amount of 15 wt% to 35 wt%, the crosslinking agent may be mixed in an amount of 1 wt% to 3 wt% based on the monomer, the thermal initiator may be mixed in an amount of 3 wt% to 6 wt% based on the monomer, and the N,N,N',N'-tetramethylethylenediamine may be mixed in an amount of 2 wt% to 4 wt% based on the monomer.

Preferably, the acrylic monomer of step i) may be included in an amount of 75 wt% to 85 wt%, and the acrylic monomer of step ii) may be included in an amount of 20 wt% to 30 wt%.

In some aspects, the acrylic monomer may be at least one selected from the group consisting of methyl methacrylate, 2-hydroxyethyl methacrylate, trimethylolpropane triacrylate, 1,4-butanediol diacrylate, ethylhexyl acrylate, glycidyl methacrylate, ethylene glycol dimethacrylate, and 1,6-hexanediol diacrylate. Preferably, the acrylic monomer may be methyl methacrylate and 2-hydroxyethyl methacrylate.

Specifically, the acrylic monomer of step i) may include 2-hydroxyethyl methacrylate and methyl methacrylate in a ratio of 8 to 10 : 1 to 2, and the acrylic monomer of step ii) may include 2-hydroxyethyl methacrylate and methyl methacrylate in a ratio of 7 to 10 : 1 to 3.

When a content of the photoinitiator of step i) is less than 0.1 wt% based on the monomer, polymerization of the monomer may not be performed, and when the content is greater than 3 wt% based on the monomer, the reaction may proceed rapidly, so that all parts of the optical unit may be polymerized, and an effect of forming a polymer-oligomer according to a UV intensity may be insufficient, so that adhesion by the photopolymerization and the thermal polymerization may not occur.

In some aspects, the photoinitiator of step i) may be a concept including any material as long as it includes a hydroxyalkyl ketone group, but preferably, may be at least one selected from the group consisting of 2-hydroxy-4'-(2-hydroxyethoxy)-2-methylpropiophenone (Irgacure 2959) , 1-hydroxy-cyclohexyl-phenyl-ketone (Irgacure 184), and bis(2,4,6-trimethylbenzoyl)-phenylphosphineoxide (Irgacure 819). More preferably, the photoinitiator of step i) may be 2-hydroxy-4'-(2-hydroxyethoxy)-2-methylpropiophenone (Irgacure 2959).

When a content of the crosslinking agent of step i) is less than 0.1 wt% based on the monomer, the optical unit may have flowability, so that it may potentially deviate from a surgical site, and a swelling degree may increase, so that it may be difficult to control a thickness or a diameter, and when the content is greater than 0.3 wt% based on the monomer, flexibility of the optical unit may be degraded, so that it may easily break, and light transmittance may decrease due to crystallization.

In some aspects, the crosslinking agent of step i) may be at least one selected from the group consisting of pentaerythritol tetraacrylate, pentaerythritol triacrylate, N,N'-methylenebisacrylamide, and ethylene glycol dimethacrylate (EGDMA). Preferably, the crosslinking agent of step i) may be ethylene glycol dimethacrylate (EGDMA).

In some aspects, the distilled water and the methanol of step i) may be mixed in a weight ratio of 75 to 95 : 5 to 25. Preferably, the distilled water and the methanol of step i) may be mixed in a weight ratio of 80 to 90 : 10 to 20.

In some aspects, the optical unit may be manufactured by only 80% to 99% through the photopolymerization in step i).

In some aspects, the distilled water and the dimethylformamide of step ii) may be mixed in a weight ratio of 70 to 90 : 10 to 30. Preferably, the distilled water and the dimethylformamide of step ii) may be mixed in a weight ratio of 75 to 85 : 15 to 25.

In some aspects, the crosslinking agent of step ii) may be at least one selected from the group consisting of pentaerythritol tetraacrylate, pentaerythritol triacrylate, N,N'-methylenebisacrylamide, and ethylene glycol dimethacrylate (EGDMA). Preferably, the crosslinking agent of step ii) may be pentaerythritol tetraacrylate.

In some aspects, the thermal initiator of step ii) may be ammonium persulfate and/or potassium persulfate. Preferably, the thermal initiator of step ii) may be ammonium persulfate.

The thermal initiator may be included in an amount of 1 wt% to 10 wt% based on the monomer, and preferably may be included in an amount of 3 wt% to 6 wt%. When the thermal initiator is less than 3 wt% based on the monomer, interfacial polymerization may not be formed, and when it is 6 wt% or more based on the monomer, the thermal initiator may affect physical properties of the support unit.

Additionally, aspects of the present disclosure may provide an artificial cornea manufactured by the manufacturing method.

In some aspects, a tensile strength of an interface between the optical unit and the support unit of the artificial cornea may be 100 KPa to 200 KPa.

In some aspects, an overall diameter of the artificial cornea may be 7 mm to 9 mm, a diameter of the optical unit may be 3 mm to 6 mm, and an apical thickness may be 0.4 mm to 0.6 mm.

In some aspects, a radius of curvature of the artificial cornea may be 7.5 mm to 8.0 mm on an anterior surface and 6.0 mm to 7.0 mm on a posterior surface.

Furthermore, aspects of the present disclosure may provide a method for manufacturing an artificial cornea consisting of an optical unit and a support unit, and the method may comprise: i) dispensing a first mixed solution comprising an acrylic monomer, a photoinitiator, a crosslinking agent, methanol, and distilled water into a lower mold, coupling an upper mold, and manufacturing the optical unit by irradiating an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine into the lower mold where the optical unit is formed, and performing thermal polymerization at 30°C to 100°C for 30 minutes to 2 hours.

In some aspects, step ii) may be continuously performed without separating the upper mold and the lower mold.

Furthermore, a mold for manufacturing an artificial cornea used in the manufacturing method may include an upper mold 10 and a lower mold 20 so that the photopolymerization and the thermal polymerization may be performed simultaneously, allowing the optical unit and the support unit of the artificial cornea to be continuously manufactured within a single mold. A first hole 11 may be formed in a central portion of the upper mold 10, a solution 12 for forming the optical unit may be inserted into the first hole 11, and a second hole 13 for introducing a thermosetting solution for forming the support unit may be formed in a peripheral portion of the upper mold 10. In addition, a plurality of lower fitting protrusions 21 for coupling with the upper mold 10 may be formed on the lower mold 20.

The upper mold 10 and the lower mold 20 may be composed of quartz and anodized aluminum. The quartz may be configured to have a radius of curvature of 6.5 mm for a lower quartz and 7.8 mm for an upper quartz, where the actual artificial cornea is manufactured, thereby implementing a curvature similar to that of a human body. A curvature characteristic of the quartz may be similarly imparted to the optical unit of the artificial cornea, so that natural integration with an originally existing cornea may be possible when inserted into the human body, and an optimized structure capable of withstanding intraocular pressure due to the human curvature may be provided.

Since the quartz is very small, measuring 4 mm to 6 mm, the addition of the anodized aluminum may facilitate handling for coupling and separating the upper mold 10 and the lower mold 20. In addition, the fitting protrusions may be formed on the peripheral portions of the upper mold 10 and the lower mold 20 to adjust a thickness of the artificial cornea to 0.5 mm.

Aluminum may have excellent corrosion resistance and oxidation resistance compared to other metals, presenting an advantage in that it may not be corroded by chemical substances. In addition, to further increase corrosion resistance and wear resistance, an anodizing coating of 25 µm may be applied.

Hereinafter, aspects of the present disclosure may be described in more detail through embodiments. It may be apparent to those skilled in the art that these embodiments may be provided solely to illustrate aspects of the present disclosure, and a scope of the present disclosure may not be construed as being limited by these embodiments.

### [Embodiment 1]

### Manufacture of an artificial cornea using a photopolymerized and thermally polymerized optical unit

### <1-1> Manufacture of an artificial cornea

Photopolymerization conditions during a manufacture of an optical unit of an artificial cornea may be investigated.

Specifically, to manufacture the optical unit, a first solution may be prepared by fixing 2-hydroxyethyl methacrylate and methyl methacrylate at a weight ratio of 10:1 and dissolving them in a first mixed solution of distilled water and methanol. At this time, the 2-hydroxyethyl methacrylate and the methyl methacrylate may be controlled to 77 wt% based on a total solution. In addition, a weight ratio of the distilled water and the methanol may be controlled to 85 parts by weight and 15 parts by weight, respectively. To the first solution, 1.2 wt% of Irgacure 2959 (2-hydroxy-4'-(2-hydroxyethoxy)-2-methylpropiophenone) and 0.12 wt% of ethylene glycol dimethacrylate (EGDMA) based on a monomer content may be added, and curing may be performed at 8 W under a 365 nm UV lamp for 2 hours.

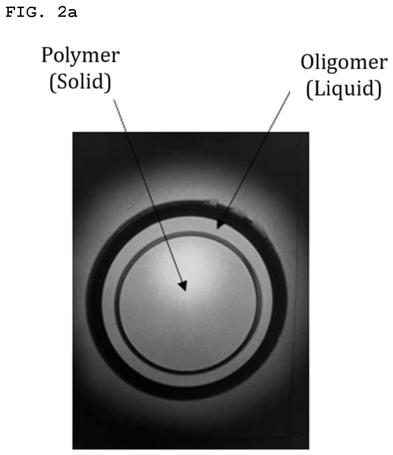

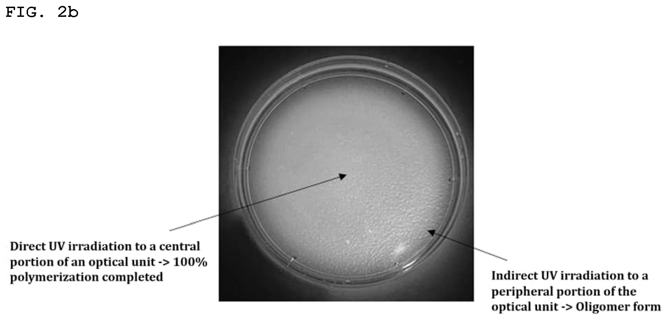

As a result of checking with a projector, when manufacturing at 8 W, it may be confirmed that photopolymerization may occur only in about 80% to 99% of the optical unit to become a solid (polymer), and a remaining 1% to 20% of the optical unit may exist as an oligomer in a liquid state (FIG. 2a and FIG. 2b).

To the optical unit manufactured at 8 W, as a support unit manufacturing solution, 80 parts by weight of 2-hydroxyethyl methacrylate and 20 parts by weight of methyl methacrylate may be dissolved in a second mixed solution of distilled water and dimethylformamide to prepare a second solution. At this time, a ratio of the two monomers may be controlled to 25 wt% based on a total capacity, and a ratio of the distilled water and the dimethylformamide, which are solvents, may be controlled to 80 parts by weight and 20 parts by weight, respectively.

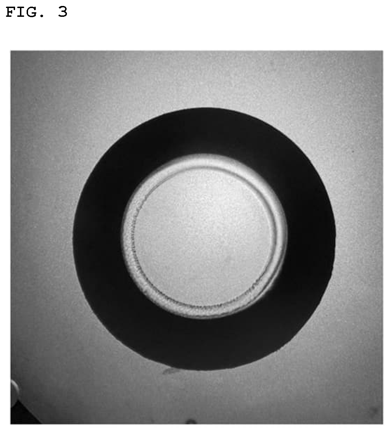

To the second solution, 1.28 wt% of a crosslinking agent pentaerythritol tetraacrylate based on the monomer, 4.6 wt% of an initiator ammonium persulfate, and 2.3 wt% of N,N,N',N'-tetramethylethylenediamine may be added, and a thermal polymerization may be performed in a 37°C dry oven for 1 hour to manufacture a polymer (FIG. 3).

### <1-2> Comparison of manufacturing times of an artificial cornea optical unit

A change in a shape of the optical unit according to a photopolymerization time may be investigated. When manufacturing the artificial cornea optical unit, the change in the shape of the optical unit may be checked by setting an irradiation time to 0 minutes, 15 minutes, 30 minutes, 1 hour, 2 hours, 4 hours, 6 hours, and 10 hours.

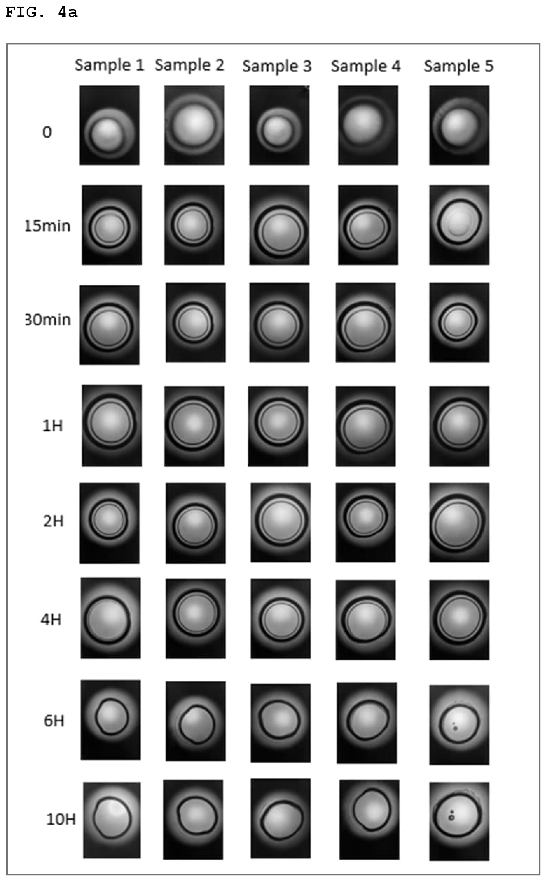

As a result, as shown in [FIG. 4a], it may be confirmed that the optical unit may be in a completely liquid state before the photopolymerization is performed. After the photopolymerization, solidification may begin to occur from 15 minutes to 30 minutes. However, as a result of manufacturing and experimenting with an artificial cornea based on a photopolymerization result, it may be confirmed that a non-polymerized portion may be washed away during a washing process, so that a central thickness of the optical unit may be less than 0.3 mm, which may be different from 0.5 mm, which is a human cornea. When a difference from 0.5 mm, which is an average thickness of the human cornea, occurs, an amount of light refraction of the cornea may be changed, so that an amount of light transmitted to a lens may be changed.

From 1 hour onwards, it may be confirmed that a thickness of a central portion of all optical units may become similar to an average thickness of a human body, being 0.486, 0.495, 0.511, 0.502, and 0.522 (mm) for sample 1 to sample 5, respectively. At this time, in an entire volume of the optical unit, a degree to which a center of the optical unit may be completely polymerized may be about 80%, and oligomerization may be about 20%. At 4 hours, a completely polymerized portion may be about 95% to 99%, and the oligomerization may be about 1% to 5%. From 6 hours, it may be confirmed that 100% polymerization of all optical units may be achieved. In addition, it may be confirmed that a deformation in shape may occur due to additional bonding between polymers.

Furthermore, in a process after 4 hours, all optical units may become polymerized, so that additional polymerization may be impossible, and physical properties of a boundary surface between the optical unit and the support unit may be reduced, so that it may be confirmed that the optical unit and the support unit may be separated during a mold extraction process (FIG. 4b) .

### [Comparative Embodiment 1]

### Manufacture of an artificial cornea using a photopolymerized optical unit

When manufacturing an optical unit, an artificial cornea may be manufactured using only photopolymerization.

Specifically, to manufacture the optical unit, a first solution may be prepared by fixing 2-hydroxyethyl methacrylate and methyl methacrylate at a weight ratio of 10:1 and dissolving them in a first mixed solution of distilled water and methanol. At this time, the 2-hydroxyethyl methacrylate and the methyl methacrylate may be controlled to 77 wt% based on a total solution. In addition, a weight ratio of the distilled water and the methanol may be controlled to 85 parts by weight and 15 parts by weight, respectively.

To the first solution, 1.2 wt% of Irgacure 2959 (2-hydroxy-4'-(2-hydroxyethoxy)-2-methylpropiophenone) and 0.12 wt% of ethylene glycol dimethacrylate (EGDMA) based on a monomer content may be added, and curing may be performed at 40 W under a 365 nm UV lamp for 2 hours.

To the optical unit manufactured at 40 W, as a support unit manufacturing solution, 80 parts by weight of 2-hydroxyethyl methacrylate and 20 parts by weight of methyl methacrylate may be dissolved in a second mixed solution of distilled water and dimethylformamide to prepare a second solution. At this time, a ratio of the two monomers may be controlled to 25 wt% based on a total capacity, and a ratio of the distilled water and the dimethylformamide, which are solvents, may be controlled to 80 parts by weight and 20 parts by weight, respectively.

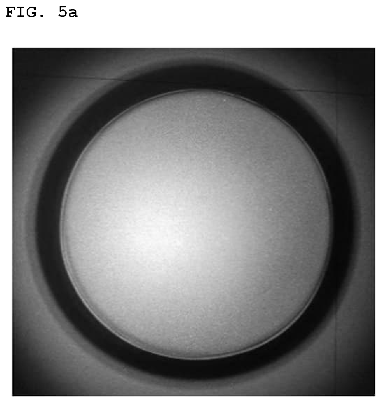

Additionally, when manufacturing at 40 W, it may be confirmed that the optical unit may be 100% polymerized (FIG. 5a).

To the second solution, 1.28 wt% of a crosslinking agent pentaerythritol tetraacrylate based on the monomer, 4.6 wt% of an initiator ammonium persulfate based on the monomer, and 2.3 wt% of N,N,N',N'-tetramethylethylenediamine based on the monomer may be added, and thermal polymerization may be performed in a 37°C dry oven for 1 hour to manufacture a polymer (FIG. 5b).

### [Comparative Embodiment 2]

### Manufacture of an artificial cornea in which an optical unit and a support unit are adhered using oxygen plasma

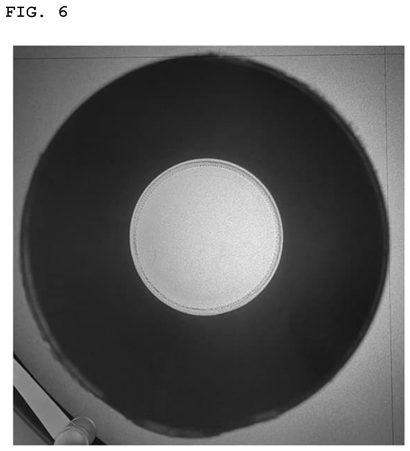

An optical unit may be manufactured by a method described in Comparative Embodiment 1 (cured for 2 hours in a 40 W 365 nm UV lamp), and a support unit may be manufactured by a method described in Embodiment 1 (80 parts by weight of 2-hydroxyethyl methacrylate and 20 parts by weight of methyl methacrylate may be dissolved in a mixed solution of distilled water and dimethylformamide to prepare a solution. At this time, a ratio of the two monomers may be controlled to 25 wt% based on a total capacity, and a ratio of the distilled water and the dimethylformamide, which are solvents, may be controlled to 90 parts by weight and 10 parts by weight, respectively. To the solution, 1.28 wt% of a crosslinking agent pentaerythritol tetraacrylate based on the monomer, 4.6 wt% of an initiator ammonium persulfate based on the monomer, and 2.3 wt% of N,N,N',N'-tetramethylethylenediamine based on the monomer may be added, and thermal polymerization may be performed in a 37°C dry oven for 1 hour to manufacture a polymer). Then, an artificial cornea may be manufactured by performing an oxygen plasma treatment for 20 minutes using a plasma system (Femto Science, Covance) which may be most generally used for interfacial adhesion (FIG. 6).

### [Embodiment 2]

### SEM image of an artificial cornea

The artificial cornea manufactured in <Embodiment 1> may be freeze-dried, and then an image may be measured at 15 KV and 1000x magnification using a mini SEM (ISP, IM-60).

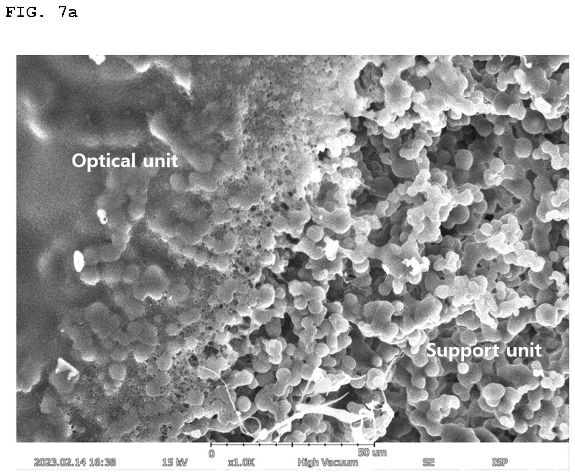

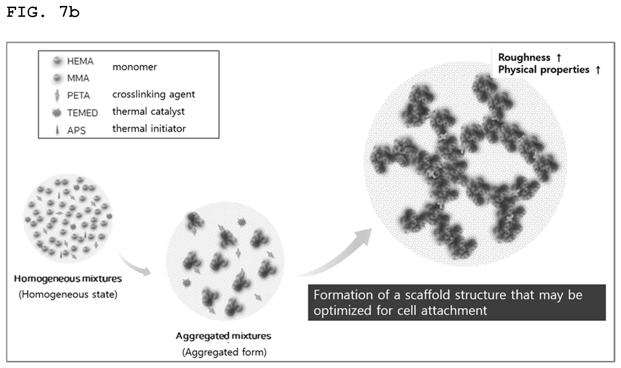

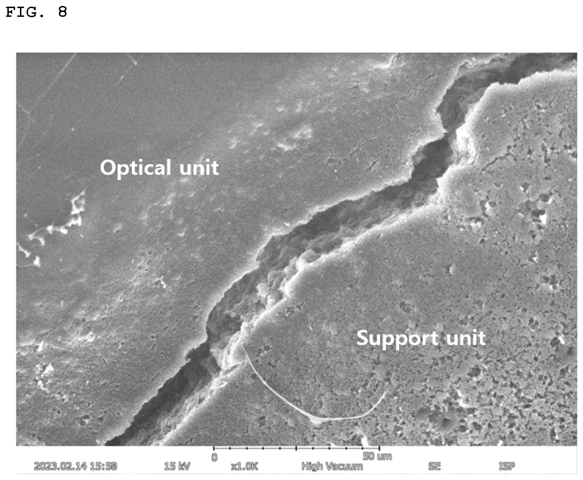

When photopolymerization and thermal polymerization are simultaneously applied to the optical unit, the support unit may be polymerized on an outer portion of the optical unit together with the oligomer, so that the support unit may easily penetrate into the oligomer, and as a result, it may be confirmed that the optical unit and the support unit may exhibit a double network form (FIG. 7a, FIG. 7b). On the other hand, when the optical unit is manufactured only by photopolymerization, the support unit solution may not penetrate into the optical unit, and polymerization may occur on an outside, so that it may be confirmed that bonding between the optical unit and the support unit may not occur properly (FIG. 8).

### [Embodiment 3]

### Confirmation of physical properties of an artificial cornea

### <3-1> Confirmation of physical properties of a boundary portion between an optical unit and a support unit

Stress-strain and tensile strength of a boundary portion between an optical unit and a support unit of artificial corneas manufactured by various methods may be investigated.

Specifically, the tensile strength of the optical unit and the support unit of Embodiment 1 and Comparative Embodiment 1, and the boundary portion between the optical unit and the support unit of the artificial corneas of Embodiment 1, Comparative Embodiment 1, and Comparative Embodiment 2 may be measured using a tensile strength meter (Instron 3345). At this time, a sample of 6 (length) * 3 (width) cm composed of the optical unit and the support unit may be used as a tensile strength sample, and a tensile strength measurement speed may be fixed at 5 mm/sec.

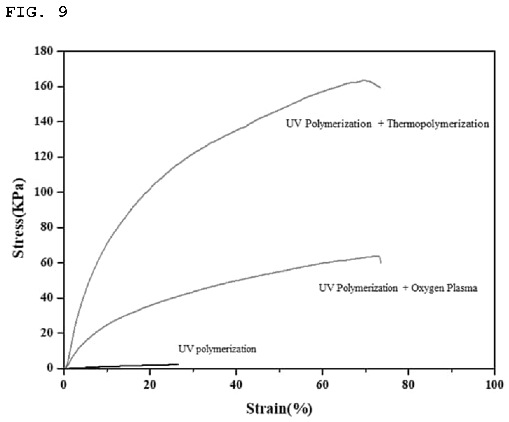

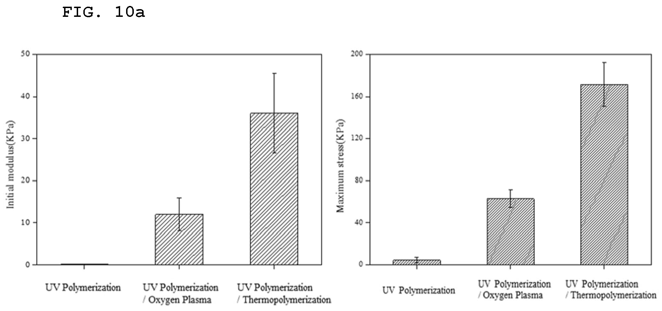

As a result, as shown in [FIG. 9] and [FIG. 10a], it may be confirmed that the stress-strain and tensile strength of the boundary portion between the optical unit and the support unit of the artificial cornea manufactured by a method of [Embodiment 1] may be highest. A maximum tensile strength may be about three times or more different from that of a method using oxygen plasma (Comparative Embodiment 2), which may be used as a most universal interfacial bonding method. In particular, when the artificial cornea is manufactured using a high energy of 40 W (Comparative Embodiment 1), it may be confirmed that a bonding strength of the boundary portion between the optical unit and the support unit may be very low, which may mean that the optical unit and the support unit may be separated by intraocular pressure after an artificial cornea surgery.

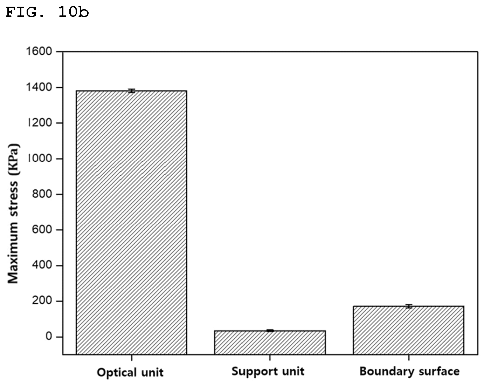

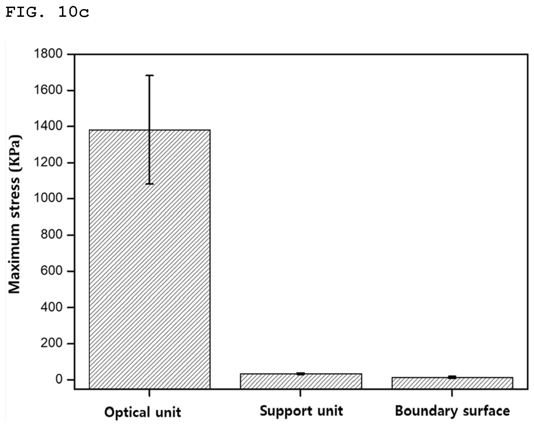

Additionally, compared to the boundary portion between the optical unit and the support unit of the artificial cornea manufactured by the method of [Embodiment 1] of the present disclosure (FIG. 10b), it may be confirmed that a relatively weak bond may occur at an interface between the optical unit and the support unit of Comparative Embodiment 1 with a maximum tensile strength of about 10 KPa (FIG. 10c). Since an average intraocular pressure of a human body may be 1.87 KPa, the support unit, which may be a contact part with the human body, may exhibit mechanical safety by bonding with an existing tissue even if it has a relatively low value, but for the optical unit and the boundary surface between the optical unit and the support unit that may not be in contact with the human body, a higher physical property may prevent detachment of the artificial cornea inserted into the human body and may prevent mechanical deformation.

### <3-2> Confirmation of physical properties of an optical unit

After manufacturing 5 optical units by the method of [Embodiment 1] or [Comparative Embodiment 1], a maximum tensile strength may be measured to confirm whether uniform polymerization may be exhibited.

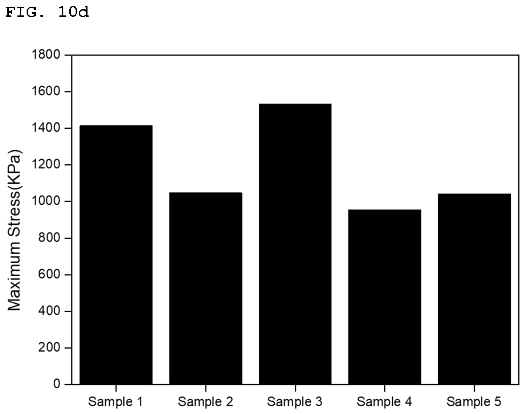

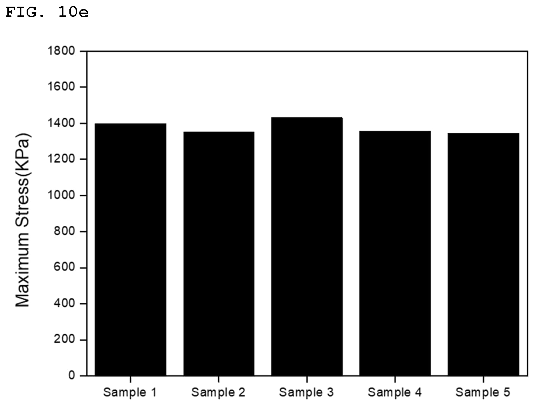

As a result, the artificial cornea manufactured by the method of [Comparative Embodiment 1] may not form uniform polymerization, so that a standard deviation of the tensile strength may be very large (FIG. 10d), whereas it may be confirmed that the artificial cornea manufactured by the method of [Embodiment 1] of the present disclosure may exhibit excellent tensile strength and bonding strength between the optical unit and the support unit through uniform polymerization (FIG. 10e). If the standard deviation of the physical properties of the optical unit is large, a safety of use may be affected.

### [Embodiment 4]

### Manufacture of an artificial cornea using a mold

An artificial cornea may be manufactured by applying an artificial cornea manufacturing method of Embodiment 1 to a mold for manufacturing an artificial cornea directly manufactured by present inventors.

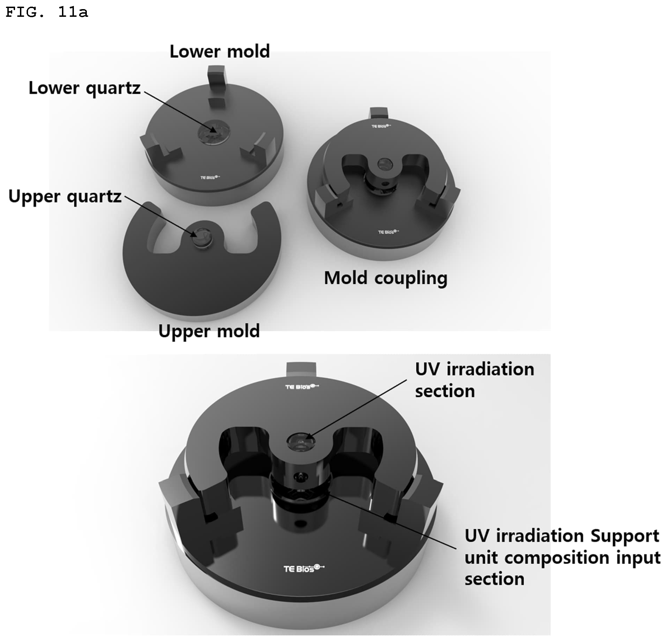

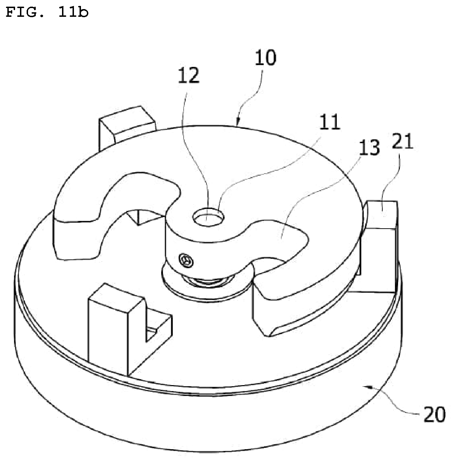

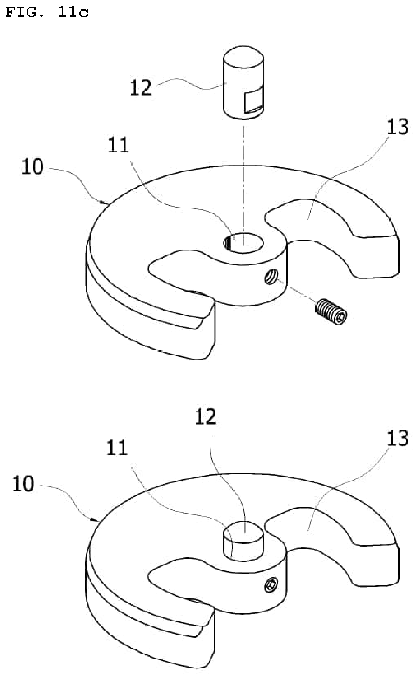

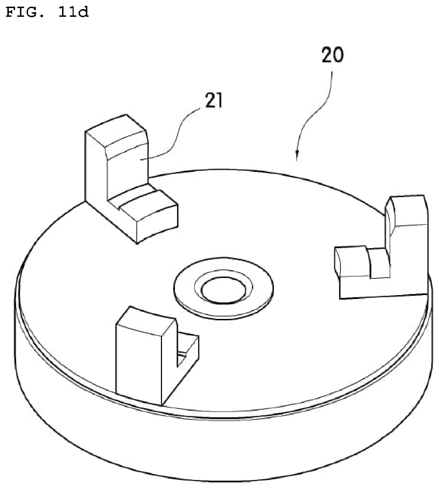

Specifically, after rough processing a base material made of an aluminum material with a 5-axis processing machine, an upper mold and a lower mold may be prepared by respectively processing them with an ultra-precision processing machine, a 350FG free-form surface processing machine from Nanotech. At this time, a first hole may be formed in a central portion of the upper mold, and a second hole for introducing a thermosetting resin composition for forming a support unit may be formed in a peripheral portion of the upper mold. Additionally, a UV transmission member made of a quartz material may be inserted into the first hole (FIG. 11a to FIG. 11d).

To a solution prepared by fixing 2-hydroxyethyl methacrylate and methyl methacrylate at a weight ratio of 10:1 and dissolving them in a mixed solution of distilled water and methanol, 1.2 wt% of Irgacure 2959 (2-hydroxy-4'-(2-hydroxyethoxy)-2-methylpropiophenone) and 0.12 wt% of ethylene glycol dimethacrylate (EGDMA) based on a monomer content may be added to manufacture a solution for forming an optical unit. 2 µL of the solution for forming the optical unit may be dispensed into a center of the lower mold (quartz) using a 20 µL pipette, and then the upper mold may be coupled, and curing may be performed at room temperature under an 8 W 365 nm UV lamp for 2 hours. At this time, the 2-hydroxyethyl methacrylate and the methyl methacrylate may be controlled to 77 wt% based on a total solution. In addition, a weight ratio of the distilled water and the methanol may be controlled to 85 parts by weight and 15 parts by weight, respectively.

Continuously, to a solution for forming a support unit prepared by dissolving 80 parts by weight of 2-hydroxyethyl methacrylate and 20 parts by weight of methyl methacrylate in a mixed solution of distilled water and dimethylformamide, 1.28 wt% of a crosslinking agent pentaerythritol tetraacrylate based on the monomer, 4.6 wt% of an initiator ammonium persulfate, and 2.3 wt% of N,N,N',N'-tetramethylethylenediamine may be added, followed by vortexing for 1 minute, and then 130 µL may be inserted into a spaced mold using a 200 µL pipette, and thermal polymerization may be performed in a 37°C dry oven for 1 hour to manufacture an artificial cornea in which the optical unit and the support unit may be integrally configured. A ratio of the two monomers may be controlled to 25 wt% based on a total capacity, and a ratio of the distilled water and the dimethylformamide, which are solvents, may be controlled to 80 parts by weight and 20 parts by weight, respectively.

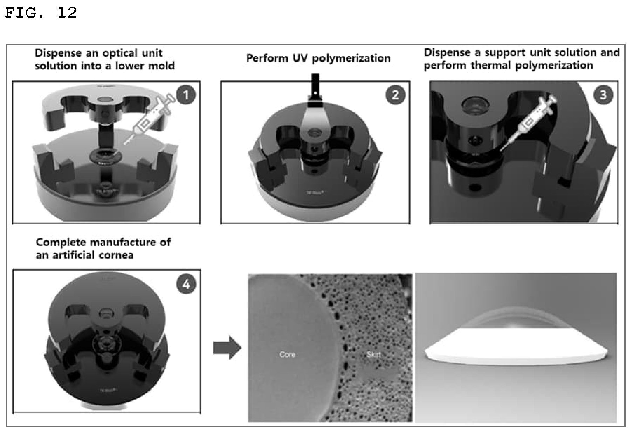

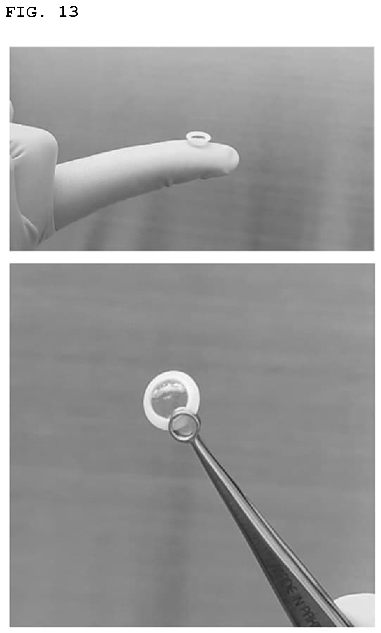

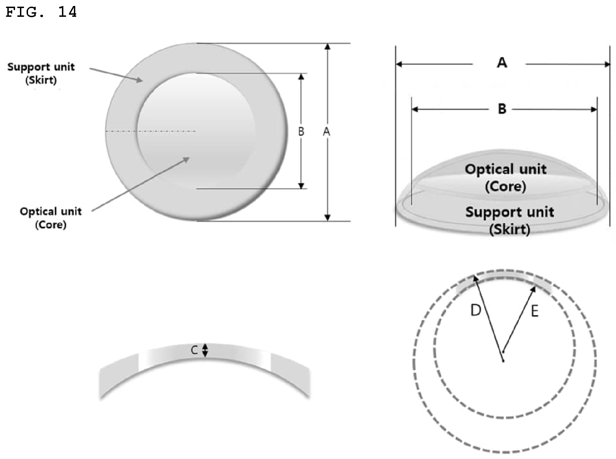

Thereafter, the mold in which a manufacture of the artificial cornea is completed may be hydrated in purified water for 24 hours, and an upper part and a lower part of the mold may be separated to obtain the artificial cornea (FIG. 12, FIG. 13). Characteristics of the manufactured artificial cornea may be as follows (FIG. 14).

**Table 1**

| **Overall Diameter (A)** | **Optical Unit Diameter (B)** | **Apical Thickness (C)** | **Radius of Curvature Anterior Surface (D)** | **Radius of Curvature Posterior Surface (E)** |
|---|---|---|---|---|
| 8 ± 0.5 mm | 4.5 ± 1 mm | 0.5 ± 0.1 mm | 7.8 ± 0.2 mm | 6.5 ± 0.2 mm |

### [Embodiment 5]

### Confirmation of cell proliferation in a manufactured artificial cornea

In vitro/in vivo cell proliferation effects of the artificial cornea manufactured by a manufacturing method of the present disclosure may be investigated.

Specifically, human fibroblasts and the artificial cornea of the present disclosure may be co-cultured and observed for 1, 3, and 7 days. In addition, the artificial cornea of the present disclosure may be implanted subcutaneously in a Rat and observed after 3 weeks and 6 weeks by H&E (hematoxylin-eosin stain) staining and M&T (Masson Trichrome stain) staining.

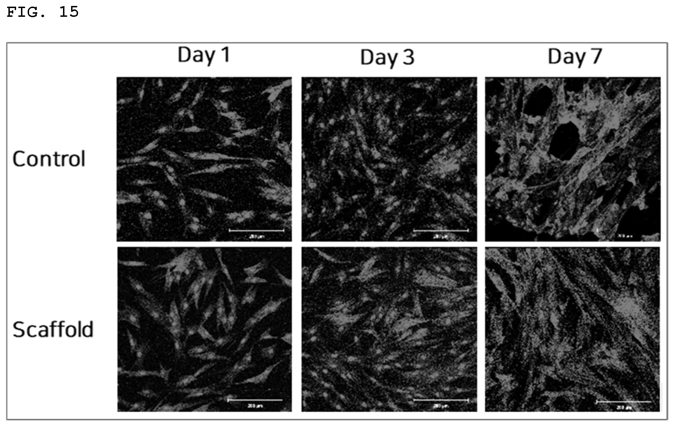

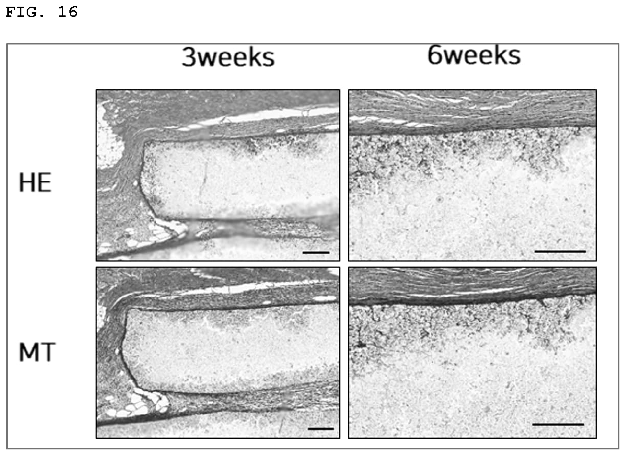

As a result, it may be confirmed that cells may penetrate into a scaffold, exhibiting excellent biocompatibility and cell proliferation (FIG. 15, FIG. 16).

### [Embodiment 6]

### Animal model experiment

### <6-1> Rabbit model

The artificial cornea manufactured by the manufacturing method of the present disclosure may be implanted into eyes of 15 NZW rabbits.

Specifically, at 4 weeks, 8 weeks, and 12 weeks after an artificial cornea implantation, a corneal surface, an implantation site, and tissue changes may be observed through Cornea H&E staining for 5 rabbits, and fibrosis may be observed using Cornea Masson's trichrome staining.

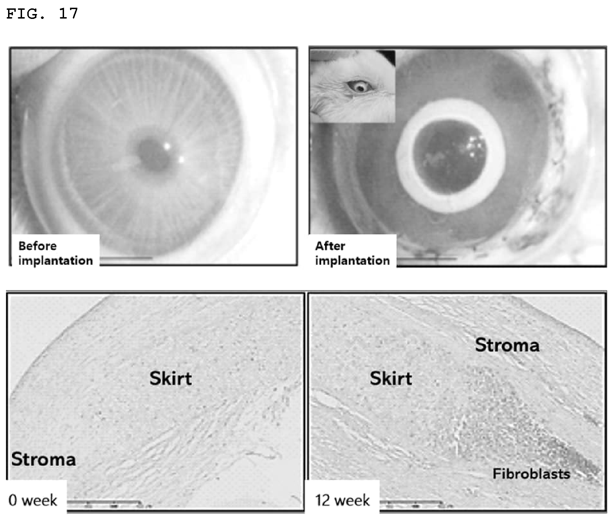

As a result, it may be confirmed that inflammation may not be expressed, there may be no visual field obstruction factors, and the support unit and corneal cells may have successfully fused (FIG. 17).

### <6-2> Primate model

Efficacy may be evaluated using following evaluation indicators for 3 months starting from 4 months after implantation in Cynomolgus monkeys.

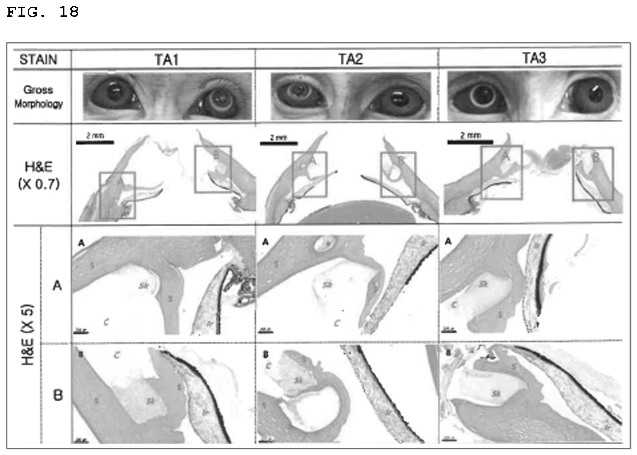

No specific findings may be observed in general symptoms and body weight changes, and as a result of a menace reflex test, no difference in response may be observed compared to an unimplanted eye. In addition, there may be no specific findings in hematological and blood biochemical tests, thereby confirming safety. As a result of histopathological examination, corneal epithelial cell proliferation and fibroblast infiltration in an implanted eye may be observed (FIG. 18).

### Industrial Applicability

According to some embodiments, an optical unit may be manufactured for 1 to 4 hours using a low-dose UV, so that an interface between the optical unit and a support unit may be stably adhered, thereby manufacturing an artificial cornea in which the optical unit and the support unit may be integrated. The artificial cornea manufactured by the manufacturing method may have a consistent spherical shape, making centering convenient and potentially increasing a surgical success rate during surgery, a central portion of the optical unit may exhibit uniform and excellent physical properties to further increase physical safety in a human body, and a boundary surface between the optical unit and the support unit may exhibit excellent physical properties of about 100 times that of existing intraocular pressure to early prevent separation of the optical unit and the support unit due to intraocular pressure in the human body. In addition, through the manufacturing method, aesthetics of a patient who has received an artificial cornea transplant may be improved, and complications and side effects caused by adhesive materials may be reduced, so there may be industrial applicability.

## Claims

1. A method for manufacturing an artificial cornea consisting of an optical unit and a support unit, the method comprising: i) manufacturing the optical unit by irradiating a first mixed solution comprising an acrylic monomer, a photoinitiator, a crosslinking agent, methanol, and distilled water with an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine to the manufactured optical unit and performing thermal polymerization at 30°C to 100°C for 30 minutes to 2 hours.

2. The method of claim 1, wherein in step i), the acrylic monomer is mixed in an amount of 70 wt% to 90 wt%, the photoinitiator is mixed in an amount of 0.1 wt% to 3 wt% based on the monomer, and the crosslinking agent is mixed in an amount of 0.1 wt% to 0.3 wt% based on the monomer.

3. The method of claim 1, wherein in step ii), the acrylic monomer is mixed in an amount of 15 wt% to 35 wt%, the crosslinking agent is mixed in an amount of 1 wt% to 3 wt% based on the monomer, the thermal initiator is mixed in an amount of 3 wt% to 6 wt% based on the monomer, and the N,N,N',N'-tetramethylethylenediamine is mixed in an amount of 2 wt% to 4 wt% based on the monomer.

4. The method of claim 1, wherein the acrylic monomer is at least one selected from the group consisting of methyl methacrylate, 2-hydroxyethyl methacrylate, trimethylolpropane triacrylate, 1,4-butanediol diacrylate, ethylhexyl acrylate, glycidyl methacrylate, ethylene glycol dimethacrylate, and 1,6-hexanediol diacrylate.

5. The method of claim 1, wherein the photoinitiator of step i) is at least one selected from the group consisting of 2-hydroxy-4'-(2-hydroxyethoxy)-2-methylpropiophenone, 1-hydroxy-cyclohexyl-phenyl-ketone, and bis(2,4,6-trimethylbenzoyl)-phenylphosphineoxide.

6. The method of claim 1, wherein the crosslinking agent of step i) is at least one selected from the group consisting of pentaerythritol tetraacrylate, pentaerythritol triacrylate, N,N'-methylenebisacrylamide, and ethylene glycol dimethacrylate (EGDMA) .

7. The method of claim 1, wherein in step i), the distilled water and the methanol are mixed in a weight ratio of 75 to 95 : 5 to 25.

8. The method of claim 1, wherein in step i), the optical unit is manufactured by only 80% to 99% through the photopolymerization.

9. The method of claim 1, wherein in step ii), the distilled water and the dimethylformamide are mixed in a weight ratio of 70 to 90 : 10 to 30.

10. The method of claim 1, wherein the crosslinking agent of step ii) is at least one selected from the group consisting of pentaerythritol tetraacrylate, pentaerythritol triacrylate, N,N'-methylenebisacrylamide, and ethylene glycol dimethacrylate (EGDMA) .

11. The method of claim 1, wherein the thermal initiator of step ii) is ammonium persulfate and/or potassium persulfate.

12. An artificial cornea manufactured by the method of claim 1.

13. The artificial cornea of claim 12, wherein a tensile strength of an interface between the optical unit and the support unit is 100 KPa to 200 KPa.

14. The artificial cornea of claim 12, wherein an overall diameter of the artificial cornea is 7 mm to 9 mm, a diameter of the optical unit is 3 mm to 6 mm, and an apical thickness is 0.4 mm to 0.6 mm.

15. The artificial cornea of claim 12, wherein a radius of curvature of the artificial cornea is 7.5 mm to 8.0 mm on an anterior surface and 6.0 mm to 7.0 mm on a posterior surface.

16. A method for manufacturing an artificial cornea consisting of an optical unit and a support unit, the method comprising: i) dispensing a first mixed solution comprising an acrylic monomer, a photoinitiator, a crosslinking agent, methanol, and distilled water into a lower mold, coupling an upper mold, and manufacturing the optical unit by irradiating an ultraviolet (UV) ray of 5 W to 40 W and 300 nm to 400 nm for 1 hour to 4 hours to perform photopolymerization; and ii) manufacturing the support unit in a state of being bonded to the optical unit by introducing a second mixed solution comprising an acrylic monomer, distilled water, dimethylformamide, a crosslinking agent, a thermal initiator, and N,N,N',N'-tetramethylethylenediamine into the lower mold where the optical unit is formed, and performing thermal polymerization at 30°C to 100°C for 30 minutes to 2 hours.

17. The method of claim 16, wherein step ii) is continuously performed without separating the upper mold and the lower mold.
